# EUROPÄISCHE PATENTSCHRIFT

(11) **EP 1 809 203 B1**
(45) Veröffentlichungstag und Bekanntmachung des Hinweises auf die Patenterteilung: **31.10.2018**
(21) Anmeldenummer: 05792405.2
(22) Anmeldetag: 16.09.2005
(51) Int. Cl.: A61F 2/91, A61F 2/915

(54) **STÜTZPROTHESE**
SUPPORTING PROSTHESIS
PROTHESE SUPPORT

(30) Priorität: 17.09.2004 DE 102004045226
(43) Veröffentlichungstag der Anmeldung: 25.07.2007
(73) Patentinhaber: Fliedner, Thilo, Dr., 82343 Pöcking (DE)
(72) Erfinder: Fliedner, Thilo, Dr., 82343 Pöcking (DE)
(74) Vertreter: Herrmann, Franz
(86) Internationale Anmeldenummer: PCT/DE2005/001635
(87) Internationale Veröffentlichungsnummer: WO 2006/029619

(56) Entgegenhaltungen:
- EP-A- 0 910 998
- WO-A-98/05270
- WO-A-2004/058104

## Beschreibung

Die Erfindung betrifft eine Stützprothese für Gefäße oder intrakorporale Lumina mit einem rohrförmigen Mantel, der ein von einem Filament gebildetes expandierbares Mäandermuster aufweist, das in einer Vielzahl von Krümmungsbögen jeweils eine Engstelle aufweist.

Eine derartige Stützprothese, ein so genannter Stent, ist aus der EP 1 374 802 A1 bekannt. Der bekannte Stent weist einen röhrenförmigen Mantel mit einer Vielzahl von mäanderförmig ausgebildeten Stützringen auf, die durch im Wesentlichen in longitudinale Richtung verlaufende, eine Vielzahl von Schlaufen aufweisende Verbindungselemente verbunden sind. Die Bogenabschnitte der mäanderförmig verlaufenden Stützringe können über den gesamten Krümmungsbogen hinweg gleichmäßig verjüngt sein, um ein Aufbiegen der Krümmungsbögen zu erleichtern. Die Krümmungsbögen dürfen jedoch nicht soweit geschwächt werden, dass die Festigkeit der Stützringe im gestreckten Zustand gefährdet ist.

Der bekannte Stent wird üblicherweise auf einen Ballonkatheter aufgebracht und mit Hilfe des Ballonkatheters in den Bereich des aufzuweitenden Gefäßes gebracht. Dies kann beispielsweise eine Stenose im Bereich eines Koronargefäßes sein. Durch Aufblasen des Ballonkatheters wird anschließend der Stent in radiale Richtung soweit expandiert, dass die Stützringe plastisch verformt werden. Durch die Stützringe wird das Gefäß auch nach dem Zurückziehen des Ballonkatheters in einem aufgeweiteten Zustand gehalten.

Bei der Expansion des Stents ist es von Vorteil, wenn die Expansion des Stents gleichmäßig in dem Sinne abläuft, dass sich die für die plastische Verformung vorgesehene Krümmungsbögen möglichst gleichzeitig gleichmäßig verformen, damit das aufzuweitende Gefäß gleichmäßig belastet wird.

Eine Stützprothese gemäß dem Oberbegriff des Anspruchs 1 ist aus der WO 2004/058104 bekannt.

Ausgehend von diesem Stand der Technik liegt der Erfindung daher die Aufgabe zugrunde, eine gleichmäßig expandierbare Stützprothese zu schaffen.
Diese Aufgabe wird durch eine Stützprothese mit den im unabhängigen Anspruch angegebenen Merkmalen gelöst. In davon abhängigen Ansprüchen sind vorteilhafte Ausgestaltungen und Weiterbildungen angegeben.
Die Stützprothese für Gefäße oder intrakorporale Lumen weist einen rohrförmigen Mantel mit einem von einem Filament gebildeten expandierbaren Mäandermuster auf, dessen Krümmungsbögen sich jeweils innerhalb des Krümmungsbogens zu einer Engstelle mit minimaler Querschnittsfläche verjüngen und dessen Krümmungsbögen durch eine von der kleinsten Querschnittsfläche zu größeren Querschnittsflächen verlaufenden Verformung streckbar sind.
Unter dem Begriff Krümmungsbogen soll ein Bogen des Mäandermusters verstanden werden, der sich zwischen geraden Abschnitten oder Wendepunkten des Mäandermusters erstreckt.
Bei einer Expansion der Stützprothese durch den Ballonkatheter werden die Mäandermuster gleichmäßig gestreckt, da bei einer Zugbelastung des Mäandermusters die Verformungen der Krümmungsbögen nahezu zeitgleich an den jeweiligen Engstellen einsetzt und sich dann kontinuierlich in die Bereiche der Krümmungsbögen fortsetzt, die einen im Vergleich zu der jeweiligen Engstelle größere Querschnittsfläche aufweisen. Insbesondere wird durch die kontinuierliche Verjüngung der Krümmungsbögen zur Engstelle hin verhindert, dass die Streckbewegung des Mäandermusters zunächst zur Streckung einzelner Krümmungsbögen führt, denn vor einer vollständigen Streckung eines Krümmungsbogen setzt die Verformung eines benachbarten Krümmungsbogens an der Engstelle ein. Auf diese Weise wird eine gleichmäßige Expansion der Stützprothese möglich.

Bei einer Ausführungsform sind die Krümmungsbögen frei von Abschnitten mit gleichbleibender Querschnittsfläche. Dadurch wird ein gleichmäßiger Streckvorgang begünstigt.

Bei einer bevorzugten Ausführungsform liegt die Engstelle abseits der Bogenmitte des jeweiligen Krümmungsbogens. Bei einer derartigen Ausgestaltung ist ein besonders großes Verhältnis von maximaler Querschnittsfläche zu minimaler Querschnittsfläche innerhalb des Krümmungsbogens herstellbar. Es ist zu erwarten, dass die Streckung des Mäandermusters bei einer Expansion des Stents besonders gleichmäßig erfolgt.

Bei einer weiteren bevorzugten Ausführungsform weist der Krümmungsbogen lediglich eine einzelne Engstelle auf. Denn bei dieser Ausgestaltung setzt die Verformung auf vorhersehbare Weise an einem definierten Ort ein und setzt sich dann in die dickeren Abschnitte des Krümmungsbogens hin fort.

Weitere Einzelheiten und Vorteile der Erfindung gehen aus der nachfolgenden Beschreibung hervor, in der Ausführungsbeispiele der Erfindung anhand der beigefügten Zeichnung im Einzelnen erläutert werden. Es zeigen:
- Figur 1: eine Aufsicht auf einen aufgeschnittenen Stent, mit einer Vielzahl von in Umfangsrichtung mäanderförmig verlaufenden Stützringen;
- Figur 2: eine vergrößerte Darstellung eines Krümmungsbogens eines der mäanderförmig verlaufenden Stützringe aus Figur 1;
- Figur 3: einen Querschnitt durch den Krümmungsabschnitt aus Figur 2 entlang der Schnittlinie III-III;
- Figur 4: einen Querschnitt durch den Krümmungsabschnitt aus Figur 2 entlang der Schnittlinie IV-IV;
- Figur 5: einen Querschnitt durch den Krümmungsabschnitt aus Figur 2 entlang der Schnittlinie V-V;
- Figur 6: eine Aufsicht auf einen weiteren aufgeschnittenen Stent mit einer Vielzahl von in Umfangsrichtung mäanderförmig verlaufenden Stützringen;
- Figur 7: eine Aufsicht auf einen aufgeschnittenen Stent, dessen mäanderförmig verlaufende Stützringe jeweils eine im Bereich der Bogenmitte der Krümmungsbögen angeordnete Engstelle aufweisen;
- Figur 8: eine vergrößerte Darstellung eines Krümmungsbogens des Stents aus Figur 7;
- Figur 9: einen Querschnitt durch den Krümmungsbogen aus Figur 8 entlang der Schnittlinie IX-IX;
- Figur 10: einen Querschnitt durch den Krümmungsbogen aus Figur 8 entlang der Schnittlinie X-X;
- Figur 11: einen Querschnitt durch den Krümmungsbogen aus Figur 8 entlang der Schnittlinie XI-XI;
- Figur 12: eine Aufsicht auf einen aufgeschnittenen Stent, bei dem das Mäandermuster einer Helixbahn folgt;
- Figur 13: eine vergrößerte Darstellung eines Krümmungsbogens eines Stents, dessen Innenbogen unterschiedliche Krümmungsradien aufweist;
- Figur 14: eine vergrößerte Darstellung eines Krümmungsbogens mit zwei Engstellen;
- Figur 15: eine vergrößerte Darstellung eines Krümmungsbogens mit zwei Engstellen, dessen Innenbogen im Bereich der Engstellen unterschiedliche Krümmungsradien aufweist; und
- Figur 16: eine vergrößerte Darstellung eines Krümmungsbogens, dessen Außenbogen eine Abflachung aufweist.

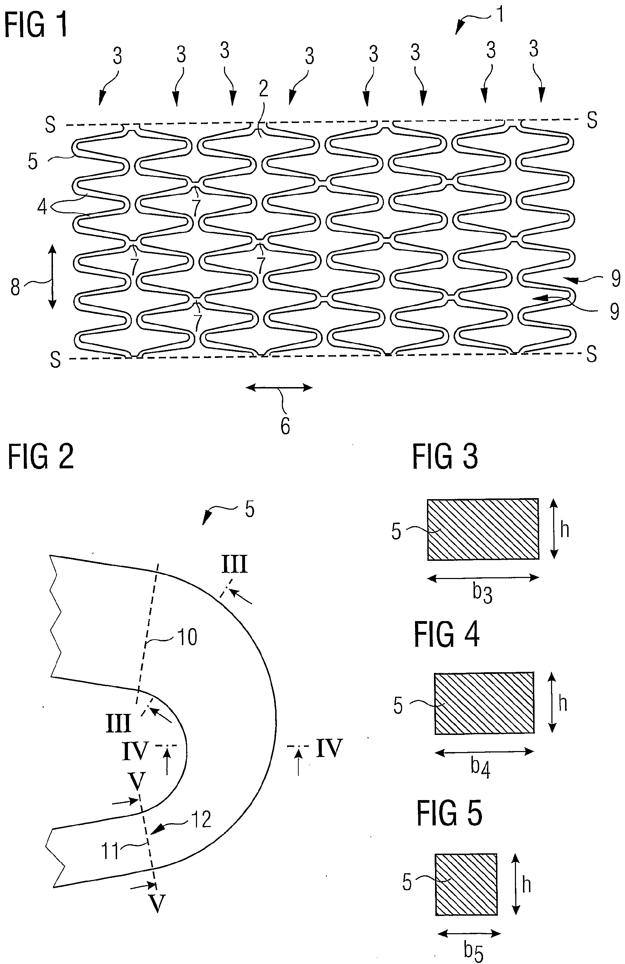

Figur 1 zeigt eine Aufsicht auf einen Stent 1 mit einem röhrförmigen Mantel 2. Der Stent 1 ist so dargestellt, wie er entlang einer Schnittlinie S-S aufgeschnitten und auf einer ebenen Fläche flach aufliegend erscheinen würde. Der Stent 1 weist eine Reihe von mäanderförmig verlaufenden Stützringen 3 auf, die sich aus einer Vielzahl von im Wesentlichen geradlinig verlaufenden Stützstreben 4 zusammensetzen, die über Krümmungsbögen 5 verbunden sind.

Die Stützringe 3 sind in eine Längsrichtung 6 durch Verbindungselemente 7 verbunden. Da der mäanderförmige Verlauf benachbarter Stützringe jeweils um 180° in der Phase versetzt ist, können die zwischen benachbarten Krümmungsbögen 5 verlaufenden Verbindungselemente 7 kurz gehalten werden. Es können jedoch auch Verbindungselemente 7 verwendet werden, die in Längsrichtung 6 einen mäanderförmigen Verlauf aufweisen. Dies ist jedoch nicht unbedingt erforderlich, da entlang den Stützringen 3 zwischen zwei Krümmungsbögen 5 mit Verbindungselementen 7 wenigstens zwei Krümmungsbögen 5 ohne Verbindungselemente 7 liegen. Zwischen den Verbindungselementen 7 kann auch jede andere gerade Zahl von Krümmungsbögen 5 ohne Verbindungselemente 7 liegen. Falls zwischen zwei Verbindungselementen 7 jeweils zwei Krümmungsbögen 5 ohne Verbindungselemente 7 liegen, weisen die Stützringe 3 zwischen jeweils zwei Verbindungselementen 7 jeweils einen N-förmigen Verlauf auf. Dadurch wird bewirkt, dass der Stent 1 auch bei einem Transport durch eng gekrümmte Gefäße ausreichend flexibel ist.

Für einen Transport durch ein Gefäß wird der röhrenförmige Stent 1 auf einen Ballonkatheter aufgebracht. Bei einer Expansion des Ballonkatheters wird der zylinderförmige Stent 1 in radiale Richtung expandiert. Dabei werden die Stützringe 3 in eine Umfangsrichtung 8 gestreckt. Die Krümmungsbögen 5 sind nun so ausgestaltet, dass die Streckbewegung möglichst gleichmäßig erfolgt. Insbesondere sollen einzelne Schlaufen 9, die von jeweils einem Krümmungsbogen 5 und den benachbarten Stützstreben 4 gebildet werden, möglichst sowohl gleichmäßig als auch zeitgleich gestreckt werden.

Unter der zeitgleichen Streckung der Krümmungsbögen 5 sind dabei Streckungen zu verstehen, die nicht notwendigerweise zur gleichen Zeit parallel ablaufen, die aber einen zeitlichen Überlapp aufweisen. Denn in der Praxis kann es aufgrund von Reibungskräften zwischen dem Stent 1 und dem Ballonkatheter vorkommen, dass die Streckung der Krümmungsbögen 5 zeitversetzt erfolgt.

In Figur 2 ist eine vergrößerte Darstellung eines der Krümmungsbögen 5 gezeigt. Die Figuren 3 bis 5 sind Querschnitte durch den Krümmungsbogen 5 aus Figur 2. Anhand der Figuren 2 bis 5 ist erkennbar, dass sich der Krümmungsbogen 5, der sich zwischen in Figur 2 gestrichelt eingezeichneten Grenzflächen 10 und 11 erstreckt, zu einer Engstelle 12 hin verjüngt.

Insbesondere anhand der Figuren 3 bis 5 ist die von der Grenzfläche 10 zur Grenzfläche 11 hin erfolgende Querschnittsreduktion deutlich erkennbar.

Vorzugsweise wird der Stent 1 aus einem Metallröhrchen gefertigt. Dabei werden die Stützringe 3 und die Verbindungselemente 7 mit Hilfe eines Lasers aus dem Metallröhrchen herausgearbeitet. So weisen die in den Figuren 3 bis 5 dargestellten Querschnitte entlang den Schnittlinien III-III, IV-IV sowie V-V jeweils die gleiche Höhe h auf. Die Querschnittsflächen unterscheiden sich jedoch hinsichtlich der Breite.

Insbesondere gilt für die jeweiligen Breiten b₃, b₄ und b₅ der Zusammenhang b₃ > b₄ > b₅.
Die Breite des Krümmungsbogens 5 kann im Bereich der Engstelle 12 soweit reduziert werden, dass die Breite b₅ des Krümmungsbogens 5 in der Engstelle 12 kleiner als die Höhe h ist.

Wie bereits erwähnt, werden bei einer Expansion des Stents 1 aufgrund einer Expansion des sich im Inneren des Stents 1 befindenden Ballonkatheters die Stützringe 3 gestreckt. Die Streckung der Stützringe 3 in Umfangsrichtung 8 wird durch eine Verformung der Krümmungsbögen 5 bewerkstelligt. Die Verformung der Krümmungsbögen 5 beginnt an der Engstelle 12, die die minimale Querschnittsfläche innerhalb des Krümmungsbogens 5 aufweist. Durch die Verformung wird der Bereich um die Engstelle 12 versprödet. Mit zunehmender Streckung der Stützringe 3 setzt sich daher die Verformung der Krümmungsbögen 5 in Richtung der Grenzfläche 10 fort. Dabei ist zunehmend größere Verformungsarbeit zu leisten. Bevor ein Krümmungsbogen 5 nahezu vollständig gestreckt wird, wird daher die Verformung eines benachbarten Krümmungsbogens 5 einsetzen. Es ist daher zu erwarten, dass die Schlaufen 9 der Stützringe 3 gleichmäßig und zeitgleich gestreckt werden.
Für das gleichmäßige Öffnen der Stützringe 3 ist es von Vorteil, wenn jeder der Krümmungsbögen 5 eine Engstelle 12 aufweist. Die Engstellen 12 sind wie in Figur 1 dargestellt bezüglich der Umfangsrichtung ausschließlich eingangsseitig oder ausgangsseitig in den jeweiligen Krümmungsbögen 5 angeordnet sein.

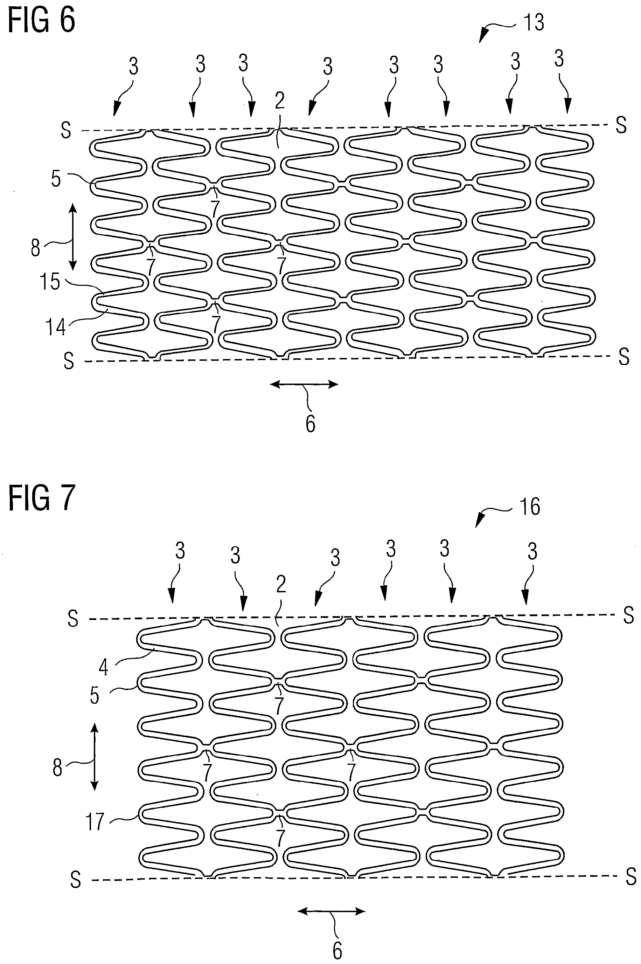

Figur 6 zeigt einen weiteren Stent 13, bei dem die Engstellen 12 abwechselnd eingangsseitig und ausgangsseitig in den Krümmungsbögen 5 angeordnet sind. Dadurch folgen in einem Stützring 3 abwechselnd breite Stützstreben 14 und schmale Stützstreben 15 aufeinander. Die schmalen Stützstreben 15 können unter Umständen federnd wirken, wenn sich Teile eines Stützrings 3 bei der Expansion in der Gefäßwand verhaken.

Figur 7 zeigt einen weiteren Stent 16, dessen Krümmungsbögen 17 jeweils eine Engstelle 18 im Bereich einer Mittellinie 19 des Krümmungsbogens 17 aufweisen.

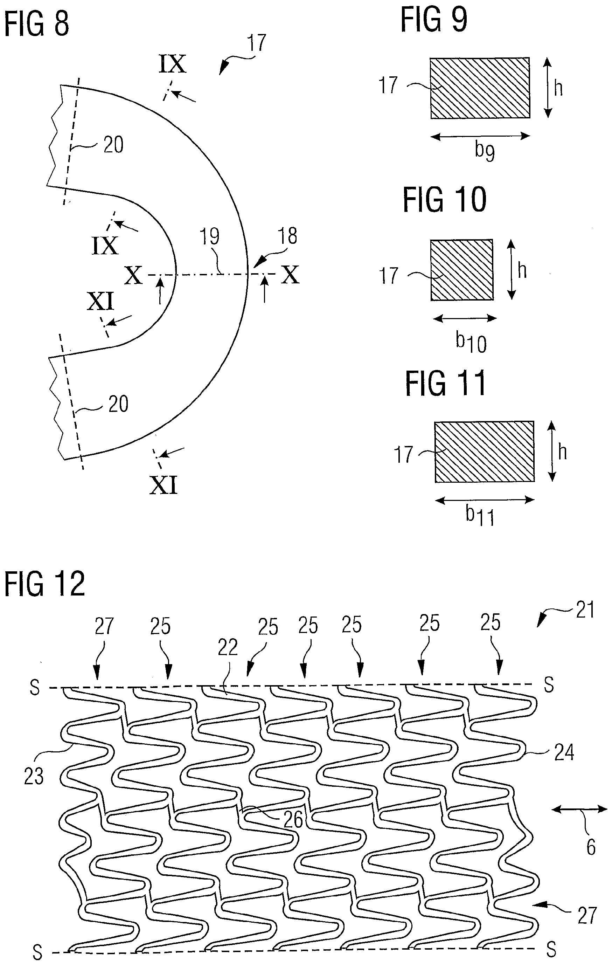

Figur 8 zeigt eine vergrößerte Darstellung eines der Krümmungsbögen 17. Die Figuren 9 bis 11 zeigen Schnitte durch den Krümmungsbogen 17 aus Figur 8 entlang den Schnittlinien IX-IX, X-X sowie XI -XI. Die Höhe h der in den Figuren 9 bis 11 dargestellten Querschnittsflächen ist jeweils gleich, lediglich die Breiten b₉, b₁₀ und b₁₁ sind unterschiedlich. Insbesondere gilt b₁₀ < b₉, b₁₁.

Bei einer Verformung des Stents 16 werden die Krümmungsbögen 17 gestreckt. Die Verformung der Krümmungsbögen 17 beginnt jeweils an der Engstelle 18 und setzt sich in beide Richtungen zu Grenzflächen 20 der Krümmungsbögen 17 hin fort. Dabei ist zunehmend größere Verformungsarbeit zu leisten, so dass die Krümmungsbögen 17 eines Stützrings 3 nahezu zeitgleich aufgebogen werden. Denn vor einer vollständigen Streckung eines der Krümmungsbögen 17 setzt die Verformung benachbarter Krümmungsbögen an der Engstelle 18 ein.

In Figur 12 ist ein entlang der Schnittlinie S-S aufgeschnittener Stent 21 dargestellt, der eine mäanderförmig verlaufende Stützhelix 22 aufweist. Die Stützhelix 22 weist dabei nahezu geradlinig verlaufende Stützstreben 23 auf, die über Krümmungsbögen 24 verbunden sind. Ferner sind nebeneinander liegende Helixbahnen 25 durch sich in Längsrichtung 6 erstreckende Verbindungselemente 26 verbunden. An den Enden des Stents 21 bildet die Stützhelix 22 jeweils Schlaufen 27.

Der Stent 21 ist ein Beispiel für einen Stent, bei dem die Stützkraft nicht von nebeneinander geordneten Stützringen, sondern von einer Stützhelix 22 aufgebracht wird. Auch bei einem Stent von der Art des in Figur 12 dargestellten Stents 21 kann das Konzept der Schwächung der Krümmungsbögen 24 verwendet werden. So können die Krümmungsbögen 24 eine den Krümmungsbögen 5 oder 17 entsprechende Gestalt aufweisen.

Auch bei der Ausgestaltung der Krümmungsbögen sind Abwandlungen möglich.

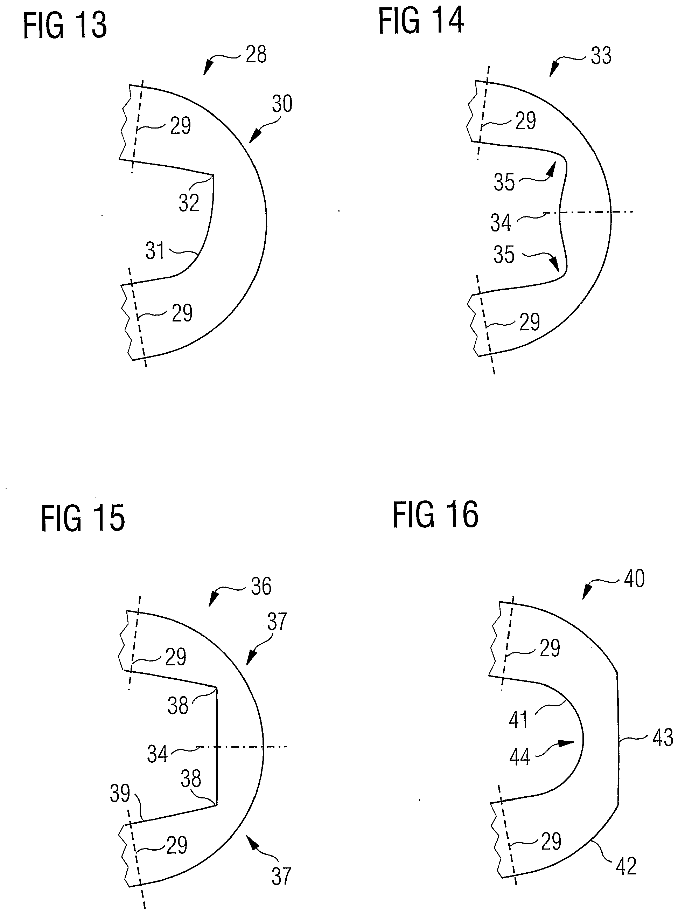

Figur 13 zeigt einen Krümmungsbogen 28, der sich zwischen Grenzflächen 29 erstreckt. Der Krümmungsbogen 28 weist eine Engstelle 30 auf, die von einer in einen Innenbogen 31 des Krümmungsbogens 28 eingebrachte Einkerbung 32 gebildet ist. In der Praxis weist die Einkerbung 32 keine scharf ausgebildete Ecke auf, da die Stents im Allgemeinen abschließend einer Elektropolitur unterzogen werden, durch die scharfe Kanten und Ecken verrundet werden. In der Praxis ist es daher so, dass die Krümmung des Innenbogens 28 im Bereich der Engstelle 32 sprungartig ansteigt.

Ein Nachteil eines Krümmungsbogens 28 von der in Figur 13 dargestellten Art ist, dass sich im Bereich der Einkerbung 32 Risse bilden können. Durch die gezielte punktuelle Schwächung wird aber andererseits erreicht, dass die Verformung der Krümmungsbögen 28 nahezu gleichzeitig erfolgt.

Figur 14 zeigt einen weiteren Krümmungsbogen 33, der zwei abseits einer Mittellinie 34 angeordnete Engstellen 35 aufweist. Der Krümmungsbogen 33 weist gewissermaßen zwei Gelenkstellen auf, die sich bei einer Verformung des Krümmungsbogens 33 verformen können.

Figur 15 zeigt einen Krümmungsbogen 36, der ebenfalls abseits der Mittellinie 34 Engstellen 37 aufweist, die durch Kerben 38 gebildet sind, die in einen Innenbogen 39 des Krümmungsbogens 36 eingebracht sind.

Figur 16 zeigt schließlich einen Krümmungsbogen 40 mit einem sich stetig krümmenden Innenbogen 41 und einem Außenbogen 42, der eine Abflachung 43 aufweist, durch die eine Engstelle 44 gebildet wird.

Die hier beschriebenen Stents 1, 13 und 16 weisen Stützringe 3 auf, deren Krümmungsbögen 5, 17, 24, 28, 33, 36 und 40 bei einer Expansion der Stents 1, 13 und 16 zeitgleich und gleichmäßig aufgebogen werden. Dadurch wird eine punktuelle Belastung der Gefäßwände vermieden.

Es sei angemerkt, dass sich die Engstellen 12 und 18 entlang einem Krümmungsbogen erstrecken können. Es ist zu erwarten, dass eine gleichmäßige Streckung der Krümmungsbögen 5 und 17 auch dann noch erfolgt, wenn sich die Engstellen 12 und 18 über etwa 25 %, in einigen Fällen auch über 50 % der Bogenlänge der Krümmungsbögen 5 und 17 erstrecken. Allerdings ist zu erwarten, dass die Streckung der Krümmungsbögen 5 und 17 besonders gleichmäßig erfolgt, wenn sich die Engstellen 12 und 18 über weniger als 10 % vorzugsweise 5 % der Bogenlänge der Krümmungsbögen 5 und 17 erstrecken.

Dementsprechend sollten sich diejenigen Abschnitte, in denen sich die Krümmungsbögen 5, 17, 24, 28, 33, 36 und 40 verjüngen, über einen möglichst großen Bereich der Krümmungsbögen 5, 17, 24, 28, 33, 36 und 40 erstrecken, um eine möglichst vollständige und gleichmäßige Streckung der Krümmungsbögen 5, 17, 24, 28, 33, 36 und 40 zu ermöglichen. Der sich verjüngende Bereich sollte sich dabei über wenigsten 50 % der Bogenlänge, vorteilhafterweise über 75 %, vorzugsweise über 90 % oder sogar über 95 % der Bogenlänge erstrecken.

Vorteilhafterweise sind die Krümmungsbögen wie in den Figurnen 1 bis 16 dargestellt frei von Abschnitten mit gleichbleibender Querschnittsfläche. Dadurch wird die gleichmäßige und zeitgleiche Streckung der Krümmungsbögen 5, 17, 24, 28, 33, 36 und 40 begünstigt. Die gleichmäßige und zeitgleiche Streckung wird ferner begünstigt, wenn wie bei dem Ausführungsbeispielen der Figuren 1 bis 12 lediglich eine einzelne Engstelle in den Krümmungsbögen 5, 17 vorhanden ist.

Unabhängig davon können die Krümmungsbögen 5, 17, 24, 28, 33, 36 und 40 bei den hier dargestellten Ausführungsbeispielen grundsätzlich vollständig gestreckt werden.

## Patentansprüche

1. Stützprothese für Gefäße oder intrakorporale Lumina mit einem rohrförmigen Mantel (2), der ein von einem Filament (3, 4, 5, 22) gebildetes expandierbares Mäandermuster aufweist, das in einer Vielzahl von Krümmungsbögen (5, 24, 28) jeweils eine Engstelle (12, 30) aufweist, zu der sich das Filament (3, 4, 5, 22) innerhalb der Krümmungsbögen (5, 24, 28) kontinuierlich hin verjüngt,
**dadurch gekennzeichnet, dass**
die Engstellen (12, 30) bei aufeinanderfolgenden Krümmungsbögen (5, 24, 28) bezüglich der Umfangsrichtung ausschließlich eingangseitig oder ausgangsseitig in den Krümmungsbögen (5, 24, 28) angeordnet sind.

2. Stützprothese nach Anspruch 1,
**dadurch gekennzeichnet, dass**
die Krümmungsbögen (5, 24, 28) frei von Abschnitten mit gleichbleibender Querschnittsfläche sind.

3. Stützprothese nach einem der Ansprüche 1 bis 2,
**dadurch gekennzeichnet, dass**
das Mäandermuster von einem in radiale Richtung expandierbaren Stützring (3) gebildet ist.

4. Stützprothese nach einem der Ansprüche 1 bis 3,
**dadurch gekennzeichnet, dass**
benachbarte Mäandermuster in der Phase um 180° versetzt angeordnet sind.

5. Stützprothese nach Anspruch 3 und 4,
**dadurch gekennzeichnet, dass**
benachbarte Mäandermuster durch Verbindungselemente (7, 26) verbunden sind, die zwischen benachbarten Krümmungsbögen (5, 24, 28) von nebeneinander angeordneten Mäandermustern angeordnet sind.

6. Stützprothese nach Anspruch 5,
**dadurch gekennzeichnet, dass**
entlang einem Mäandermuster zwischen Krümmungsbögen (5, 24, 28) ohne Verbindungselemente (7) eine gerade Anzahl von Krümmungsbögen (5, 24, 28) mit Verbindungselementen (7, 26) liegt.

7. Stützprothese nach einem der Ansprüche 1 bis 6,
**dadurch gekennzeichnet, dass**
das Mäandermuster wellenförmig ausgebildet ist.

8. Stützprothese nach einem der Ansprüche 1 bis 2,
**dadurch gekennzeichnet, dass**
das Mäandermuster einer Helixbahn folgt.

9. Stützprothese nach Anspruch 8,
**dadurch gekennzeichnet, dass**
das Filament, das das Mäandermuster bildet, an den Enden des rohrförmigen Mantels jeweils eine geschlossene Schlaufe (27) bildet.

10. Stützprothese nach einem der Ansprüche 1 bis 9,
**dadurch gekennzeichnet, dass**
die Krümmung des Innenbogens und des Außenbogens innerhalb des Krümmungsbogens einen stetigen Verlauf aufweist.

## Claims

1. Support prosthesis for vessels or intracorporeal lumens with a tubular casing (2) having an expandable meandering pattern which is formed by a filament (3, 4, 5, 22) and has a respective narrowing (12, 30) in a number of bending curves (5, 24, 28), wherein the filament (3, 4, 5, 22) tapers within the bending curves (5, 24, 28) continuously toward each narrowing (12, 30),
**characterised in that**
the narrowings (12, 30) in adjacent bending curves (5, 24, 28) are arranged with respect to the circumferential direction exclusively on the inlet side or the outlet side in the respective bending curves (5, 24, 28).

2. Support prosthesis according to claim 1,
**characterised in that**
the bending curves (5, 24, 28) are free from portions having a constant cross-sectional area.

3. Support prosthesis according to any one of claims 1 to 2,
**characterised in that**
the meandering pattern is formed by a support ring (3) expandable in the radial direction.

4. Support prosthesis according to any one of claims 1 to 3,
**characterised in that**
adjacent meandering patterns are arranged phase-offset through 180°.

5. Support prosthesis according to any one of claims 3 and 4,
**characterised in that**
adjacent meandering patterns are connected by connecting elements (7, 26) arranged between adjacent bending curves (5, 24, 28) of meandering patterns arranged next to one another.

6. Support prosthesis according to claim 5,
**characterised in that**
an even number of bending curves (5, 24, 28) with connecting elements (7, 26) are located along a meandering pattern between bending curves (5, 24, 28) without connecting elements (7).

7. Support prosthesis according to any one of claims 1 to 6,
**characterised in that**
the meandering pattern is undulating in its configuration.

8. Support prosthesis according to any one of claims 1 to 2,
**characterised in that**
the meandering pattern follows a helix path.

9. Support prosthesis according to claim 8,
**characterised in that**
the filament forming the meandering pattern forms a respective closed loop (27) at the ends of the tubular casing.

10. Support prosthesis according to any one of claims 1 to 9,
**characterised in that**
the curvature of the inner curve and the outer curve within the bending curve has a continuous course.

## Revendications

1. Prothèse d'appui pour vaisseaux ou lumières intracorporelles avec une enveloppe tubulaire (2) qui présente un modèle en méandres pouvant être dilaté, formé d'un filament (3, 4, 5, 22), qui présente à chaque fois un rétrécissement (12, 30) dans une pluralité d'arcs de courbure (5, 24, 28) vers lequel le filament (3, 4, 5, 22) se rétrécit en continu au sein des arcs de courbure (5, 24, 28),
**caractérisée en ce que**
les rétrécissements (12, 30) sont disposés dans les arcs de courbure (5, 24, 28) exclusivement du côté de l'entrée ou du côté de la sortie par rapport à la direction périphérique dans le cas d'arcs de courbure successifs (5, 24, 28).

2. Prothèse d'appui selon la revendication 1,
**caractérisée en ce que**
les arcs de courbure (5, 24, 28) sont exempts de sections avec une superficie de la section constante.

3. Prothèse d'appui selon une des revendications 1 à 2,
**caractérisée en ce que**
le modèle en méandres est formé d'un anneau d'appui (3) pouvant être dilaté dans la direction radiale.

4. Prothèse d'appui selon une des revendications 1 à 3,
**caractérisée en ce que**
des modèles en méandres voisins sont disposés de manière déphasée de 180°.

5. Prothèse d'appui selon les revendications 3 et 4,
**caractérisée en ce que**
des modèles en méandres voisins sont connectés par des éléments de connexion (7, 26) qui sont disposés entre des arcs de courbure voisins (5, 24, 28) de modèles en méandres disposés côte à côte.

6. Prothèse d'appui selon la revendication 5,
**caractérisée en ce que**
un nombre pair d'arcs de courbure (5, 24, 28) avec des éléments de connexion (7, 26) se situe le long d'un modèle en méandres entre des arcs de courbure (5, 24, 28) sans éléments de connexion (7).

7. Prothèse d'appui selon une des revendications 1 à 6,
**caractérisée en ce que**
le modèle en méandres est réalisé de forme ondulée.

8. Prothèse d'appui selon une des revendications 1 à 2,
**caractérisée en ce que**
le modèle en méandres suit un trajet hélicoïdal.

9. Prothèse d'appui selon la revendication 8,
**caractérisée en ce que**
le filament qui forme le modèle en méandres forme aux extrémités de l'enveloppe tubulaire à chaque fois une boucle fermée (27).

10. Prothèse d'appui selon une des revendications 1 à 9,
**caractérisée en ce que**
la courbure de l'arc interne et de l'arc externe présente une allure continue au sein de l'arc de courbure.
